# EUROPÄISCHE PATENTSCHRIFT

(11) **EP 0 462 608 B1**
(45) Veröffentlichungstag und Bekanntmachung des Hinweises auf die Patenterteilung: **11.09.1996**
(21) Anmeldenummer: 91110115.2
(22) Anmeldetag: 20.06.1991
(51) Int. Cl.: A61L 25/00, A61K 9/22

(54) **Implantierbares Wirkstoffdepotmaterial**
Implantable drug depot material
Matériau implantable pour dépôt médicamenteux

(30) Priorität: 20.06.1990 DE 4019617
(43) Veröffentlichungstag der Anmeldung: 27.12.1991
(73) Patentinhaber: THERA Patent GmbH & Co. KG Gesellschaft für industrielle Schutzrechte, D-82229 Seefeld (DE)
(72) Erfinder: Stille, Wolfgang, Prof. Dr., W-6000 Frankfurt/Main (DE); Gentschew, Gabriele, Dr., W-6000 Frankfurt/Main (DE); Reitz, Peter, W-6053 Obertshausen (DE); Erben, Thomas, W-6200 Wiesbaden (DE); Zöllner, Werner, Dr., W-8031 Wörthsee/ Steinebach (DE); Stefan, Klaus-Peter, Dr., W-8031 Seefeld (DE)
(74) Vertreter: Abitz, Walter, Dr.-Ing.

(56) Entgegenhaltungen:
- EP-A- 0 202 445
- EP-A- 0 331 071
- EP-A- 0 357 327

## Beschreibung

Es ist bekannt, daß immer häufiger systemische durch lokale Therapien ersetzt werden, da auf diese Weise in schwer erreichbaren Körperregionen hohe Wirkstoffspiegel erzielt werden, ohne daß über den gesamten Körper verteilte hohe, oft toxische Chemotherapeutikadosen appliziert werden müssen.

Die DE-C-20 22 117, 25 11 122 und 27 27 535 beschreiben Knochenzemente auf Polymethylmethacrylat(PMMA)-Basis, die ein Antibiotikum wie Gentamycin enthalten. Daneben sind weitere antibiotikahaltige Knochenzemente auf PMMA-Basis bekannt. So beschreiben beispielsweise die EP-A-0 301 759 PMMA-Knochenzemente, die Erythromycin enthalten und die DE-A-35 42 972 und 35 43 164 PMMA-Knochenzemente, die sogenannte Gyrasehemmer enthalten. Es ist ebenfalls bekannt, Implantate auf Basis oben genannter PMMA-Knochenzemente in vorgefertigter Form zur lokalen Antibiotikatherapie einzusetzen, wozu beispielsweise auf die DE-C-26 51 441 und 27 27 535 verwiesen wird.

Auch die Freisetzung von Gyrasehemmern aus keramischen Trägermaterialien wie Tricalciumphosphat ist beschrieben, beispielsweise in der DE-A-35 42 972.

Weiterin ist bekannt, Zytostatika, beispielsweise Methotrexat, "Knochenzementen" auf PMMA-Basis zuzusetzen (siehe z.B. H. Wahlig, E. Dingeldein in "Primär- und Revisionsalloarthroplastik", Herausgeber: Endo-Klinik, Hamburg, Springer-Verlag, Berlin 1987, Seite 357).

In der Dentalmedizin werden seit Anfang der 70er Jahre Glasionomerzemente als Füllungsmaterialien sowie zum Befestigen von Kronen und Brücken eingesetzt (z.B. DE-A-20 61 513). Hierbei erhärten die Materialien durch Reaktion des basischen Glaspulvers mit einer polymeren Polysäure unter Bildung einer hochpolaren Calcium-Aluminium-Polysalzmatrix. Ihre Verwendung als Knochenzement ist ebenfalls bekannt, (siehe z.B. L. M. Jonck, C. J. Grobbelaar, H. Strating, Clin. Mat. 4, 85 (1989)). Schließlich ist es bekannt, Glasionomerzemente in poröser, geschäumter Form als Knochenersatzmaterial einzusetzen (siehe z.B. DE-A-38 06 448).

Die Verwendung von PMMA-Knochenzementen als Wirkstoffdepot birgt verschiedene Nachteile. Z.B. enthalten die Materialien auch in ausgehärteter Form noch deutliche Mengen an gesundheitsschädlichen Monomeren, insbesondere Methylmethacrylat, sowie nicht abreagierte Peroxide und andere Radikalbildner. Beim Einbringen des nicht gehärteten Materials zu Zementierungszwecken wird bei der Abbindung eine Abbindetemperatur von bis zu 80°C erreicht, was das direkt angrenzende Körpergewebe zerstören kann. Die Wirkstofffreisetzung aus solchen Knochenzementen und daraus hergestellten Implantaten ist nur bei einigen ausgewählten Vertretern einigermaßen befriedigend. Wünschenswert ist eine anfänglich hohe Freisetzung, gefolgt von einer nachträglichen kontinuierlichen Freigabe geringerer Dosen innerhalb von 2 bis 30 Tagen. Nach diesem Zeitpunkt wird in idealer Weise kein weiterer Wirkstoff abgegeben. Dies konnte mit Wirkstoffdepots auf Basis PMMA bisher nicht befriedigend erreicht werden.

Die EP-A-0 357 327 beschreibt implantierbare Zementmaterialien zur kontrollierten verzögerten Freisetzung von Wirkstoffen. In Verbindung mit einer Reihe von Wirkstoffen wird über erhebliche Schwierigkeiten bei der Abbindung oder hinsichtlich der Zersetzung der Wirkstoffe berichtet. Zytostatika und Antibiotika werden als Wirkstoffe nicht in Betracht gezogen und nicht erwähnt.

Aufgabe der vorliegenden Erfindung ist es, oben genannte Nachteile des Standes der Technik zu vermeiden und Wirkstoffdepots zur Verfügung zu stellen, die ein optimales Wirkstoffabgabespektrum ermöglichen. Überraschenderweise hat sich gezeigt, daß die Verwendung von Glasionomerzementen in Verbindung mit Antibiotika bzw. Zytostatika eine ideale Kombination ergibt. Zum einen lassen sich hiermit hohe anfängliche Freisetzungsraten erzielen, insbesondere auch bei Vertretern aus der Gruppe der Antibiotika, wie Clindamycin, welche bei PMMA keine befriedigenden Freisetzungsraten ergeben, und zum anderen erzielt man anschließend eine Abgabe des Wirkstoffs in geringen Mengen bis nahezu kein Wirkstoff mehr abgegeben wird. Dies ist umso überraschender, als hierbei in der Regel polare Wirkstoffe aus einer extrem polaren Matrix freigesetzt werden. An sich würde man erwarten, daß die Wirkstoffe in der Glasionomerzementmatrix gut festgehalten werden.

Gegenstand der Erfindung ist ein implantierbares Wirkstoffdepotmaterial, das einen Glasionomerzement und ein oder mehrere Zytostatika oder Antibiotika enthält. Vorzugsweise enthalten die erfindungsgemäßen Wirkstoffdepots die folgenden Komponenten:
(a) ein Aluminiumfluorosilikatglas,
(b) mindestens eine Polysäure mit einem mittleren Molekulargewicht > 500,
(c) gegebenenfalls Wasser,
(d) ein oder mehrere Zytostatika oder Antibiotika,
(e) gegebenenfalls ein Carbonat und/oder Hydrogencarbonat in einer Menge von mindestens 0,1 Gew.-%, bezogen auf (a), oder ein anderes Verschäumungsmittel, und
(f) gegebenenfalls einen Chelatbildner.

In einer bevorzugten Ausführungsform besteht das erfindungsgemäße Material aus einer flüssigen Teilmasse, die 40 bis 90 Gew.-% Wasser, 10 bis 60 Gew.-% Polysäure sowie 0 bis 20 Gew.-% Chelatbildner enthält, und aus einer festen Teilmasse, die 80 bis 99,89 Gew.-% Aluminiumfluorosilikatglas, 0,01 bis 3 Gew.-% eines oder mehrerer Zytostatika oder Antibiotika und gegebenenfalls 0,1 bis 20 Gew.-% eines Carbonats und/oder Hydrogencarbonats enthält.

In einer weiteren bevorzugten Ausführungsform besteht das erfindungsgemäße Material aus einer flüssigen Teilmasse, die 80 bis 100 Gew.-% Wasser sowie 0 bis 20 Gew.-% Chelatbildner enthält, und aus einer festen Teilmasse, die 50 bis 94,89 Gew.-% Aluminiumfluorosilikatglas, 5,0 bis 49,98 Gew.-% trockene Polysäure, 0,01 bis 3 Gew.-% eines oder mehrerer Zytostatika oder Antibiotika und gegebenenfalls 0,1 bis 20 Gew.-% Carbonat und/oder Hydrogencarbonat enthält.

Zusätzlich können Stabilisatoren, Konservierungsmittel, wie z.B. Benzoesäure, Thixotropiehilfsmittel, Desinfektionsmittel, Pigmente, Röntgenkontrastmittel und weitere Füllstoffe enthalten sein.

Die erfindungsgemäßen Wirkstoffdepotmaterialien liegen in plastischer Form zu Zementierungszwecken oder aber vorzugsweise abgebunden in Form von vorgefertigten Formkörpern, insbesondere in Form von Granulaten vor, wobei geschäumte Formkörper bevorzugt sind.

Bei Einsatz in plastischer Form werden die Chemotherapeutika den anderen Komponenten zugemischt. Eine Anwendungsform kann in vorgemischten Einzelbestandteilen bestehen. Die sechs Bestandteile (a) - (f) können beispielsweise folgendermaßen auf zwei Komponenten A und B verteilt werden.

| | A | B |
|---|---|---|
| 1 | a + b + d | c |
| 2 | a + b | c + d |
| 3 | a + d | b + c |
| 4 | a | b + c + d |

Der Chelatbildner (f) kann jeder der Komponenten A oder B zugemischt werden, während sich das Verschäumungsmittel (e) vorzugsweise in der Komponente befindet, die keine Säure (b) oder (f) enthält (diese Einschränkung gilt nicht für trockene Zusammensetzungen).

Gibt man zur Verarbeitung des erfindungsgemäßen Materials feste und flüssige Teilmassen zusammen, so entsteht durch die Reaktion der Polysäure mit dem Aluminiumfluorosilikatglas ein Formkörper, welcher durch die gleichzeitige Reaktion der Polysäure mit dem Carbonat und/oder Hydrogencarbonat mit interkonnektierenden Makroporen versehen wird.

Das Einbringen der Zytostatika oder Antibiotika in die Formkörper kann beispielsweise in der plastischen Phase, wie oben geschildert, geschehen oder aber auch durch Tränken von vorher hergestellten Glasionomerzementformkörpern, vorzugsweise geschäumten Formkörpern, mit den Zytostatika oder Antibiotika in einem geeigneten Lösungsmittel, welches physiologisch unbedenklich sein sollte. Das Lösungsmittel kann vor Implantation der Formteile auch in üblicher Weise entfernt werden. Die Formkörper haben vorzgusweise Granulat- oder Kugelform und eine maximale Korngröße ≦ 20 mm, bevorzugt ≦ 5 mm; besonders bevorzugt sind Granulate der Korngrößen 0,5 - 1, 1 - 2, 2 - 3 und 3 - 4 mm. Die Formkörper können einzeln oder auch als Ketten analog den bekannten PMMA-Ketten der DE-C-26 51 441 als Wirkstoffdepot verwendet werden. Auch eine Kombination von Wirkstoffdepots auf Glasionomerzement-Basis und auf PMMA-Basis ist für spezielle Indikationen möglich.

Mögliche Indikationsgebiete der erfindungsgemäß hergestellten Massen sind z.B. antibiotikahaltige Zemente zum Befestigen künstlicher Gelenke, zur Rekonstruktion knöcherner Defekte, für retrogade Wurzelfüllungen im Rahmen einer Wurzelspitzenresektion, zur Zementierung eines apikalen Titan- oder Goldstiftes im Rahmen einer Wurzelspitzenresektion, zur Zementierung eines Guttaperchastiftes im Rahmen einer orthograden Wurzelfüllung oder zur Pulpitisbehandlung als Zahnfüllungsmaterial.

Zytostatikahaltige Zemente können beispielsweise zum Auffüllen von Knochenhöhlen nach chirurgischen Tumorentfernungen eingesetzt werden.

Antibiotikahaltige Formkörper oder Granulate können zur Rekonstruktion knöcherner Defekte eingesetzt werden, z.B. im Schädelbereich, zum Auffüllen infizierter Mastoidhöhlen, zum Auffüllen großer Defekte bei Hüftpfannenrevisionen sowie im Rahmen der konservierenden Zahnheilkunde als Bestandteil einer Unterfüllung zur Behandlung einer tiefen Karies oder als Formteil oder Kette bei Knochen- und/oder Weichteilinfektionen eventuell in Verbindung mit den bereits bekannten PMMA-Ketten.

Zytostatikahaltige Formteile oder Granulate können vorteilhaft zur Rekonstruktion und/oder Auffüllung von Knochendefekten nach chirurgischer oder cryochirurgischer Tumorentfernung eingesetzt werden.

Auch die Kombination von vorgefertigten Formteilen und plastisch eignebrachtem Zement kann sinnvoll sein. Je nach Indikationsstellung können hierbei die Formteile und Zemente gleiche oder unterschiedliche Wirkstoffe und/oder Wirkstoffkombinationen enthalten.

Die mit dem erfindungsgemäßen Material herstellbaren porösen Formkörper können vor ihrer Verwendung an der Oberfläche leicht angeschliffen werden, so daß die erzielten Poren frei zugänglich sind.

Mit den erfindungsgemäßen Materialien lassen sich die Vorteile gut biokompatibler Materialien, wie Hydroxylapatitkeramiken, mit der guten Applizierbarkeit von gefüllten Polyacrylaten verbinden, ohne hierbei die Nachteile, wie Zusatz von resorbierbaren Substanzen, Einsatz von starken Säuren, Einsatz von toxikologisch bedenklichen Monomeren und schwierige Formgebung harter Keramiken und Gläser, in Kauf nehmen zu müssen.

Mit den Materialien gemäß der Erfindung lassen sich für den Einsatz als Knochenersatz pastöse Massen anmischen, die vom Chirurgen während der Operation einfach und ohne Bearbeitung mit spanabhebenden Werkzeugen in die gewünschte Form gebracht werden können oder direkt in die Knochendefekte eingefüllt werden können, wobei dann die Erhärtung im Knochen erfolgt. Es werden gut verträgliche Knochenersatzteile erhalten, die aufgrund ihrer Porosität vom Knochenmaterial gut durchwachsen werden können und bei der Abbindereatkion weder durch Säureeinwirkung noch durch Temperaturspitzen das umgebende Knochenmaterial schädigen.

Durch die Korngröße und Menge der eingesetzten Carbonate und/oder Hydrogencarbonate und durch die Wahl der Löslichkeit der Carbonate und der eingesetzten Polysäure läßt sich die Makroporosität beeinflussen. Es lassen sich so in idealer Weise Knochenersatzteile herstellen, die in ihrer Porosität dem umgebenden Knochenmaterial, insbesondere der Spongiosa, gleichen.

Die Einzelkomponenten der erfindungsgemäßen Materialien können vordosiert in Ausbringvorrichtungen (z.B. Kapseln), wie sie beispielsweise in der DE-A-34 07 648 beschrieben sind, angewendet werden. In diesen Ausbringvorrichtungen befindet sich die pulverförmige Komponente in der Regel im Kapselinnenraum, während sich die flüssige Komponente in einem Kissen an der Kapselwand befindet. Vor der Anwendung wird - mit einem speziellen Aktivator - der Inhalt der Kissenflüssigkeit durch ein Loch in der Kapselwand ins Kapselinnere gepreßt. Durch Schütteln wird der Inhalt homogen vermischt. Das Material kann aus der Kapsel direkt in die Knochenöffnung eingebracht werden. Bei dieser Anwendungsweise ist es vorteilhaft, wenn Glas und Carbonat und/oder Hydrogencarbonat als Pulver im Kapselinnenraum aufbewahrt werden und die wässrige Polysäurelösung und gegebenenfalls der Chelatbildner als Flüssigkeitskomponente im Kissen enthalten sind.

Als Bestandteil (a) können die in der DE-A-20 61 513 und der EP-A-0 023 013 beschriebenen Calciumaluminiumfluorosilikatgläser und die in der EP-A-0 241 277 beschriebenen Strontiumaluminiumfluorosilikatgläser eingesetzt werden. Die erfindungsgemäß verwendeten Aluminiumfluorosilikatglas-Pulver bestehen neben Sauerstoff vorzugsweise aus:

| Bestandteil | Berechnet als | Gew.-% |
|---|---|---|
| Si | SiO₂ | 20 - 60 |
| Al | Al₂O₃ | 10 - 50 |
| Ca | CaO | 0 - 40 |
| Sr | SrO | 0 - 40 |
| F | F | 1 - 40 |
| Na | Na₂O | 0 - 10 |
| P | P₂O₅ | 0 - 10 |

wobei mindestens 1 Gew.-% CaO und/oder SrO enthalten sein müssen und insgesamt 0 bis 20 Gew.-%, berechnet als Oxide, an B, Bi, Zn, Mg, Sn, Ti, Zr, La oder anderen 3-wertigen Lanthanoiden, K, W, Ge sowie weiteren Zusätzen, welche die Eigenschaften nicht beeinträchtigen und physiologisch unbedenklich sind. Durch Zusatz von 10 bis 20 Gew.-% La₂O₃ können die Gläser röntgensichtbar gemacht werden.

Vorteilhaft bestehen die Pulverteilchen aus:

| | |
|---|---|
| Si als SiO₂ | 25 - 50 Gew.-% |
| Al als Al₂O₃ | 10 - 40 Gew.-% |
| Ca als CaO | 0 - 35 Gew.-% |
| Sr als SrO | 0 - 35 Gew.-% |
| F | 5 - 30 Gew.-% |
| Na als Na₂O | 0 - 8 Gew.-% |
| P als P₂O₅ | 1 - 10 Gew.-% |

wobei mindestens 10 Gew.-% Ca (berechnet als CaO) und/oder Sr (berechnet als SrO) enthalten sein müssen und 0 - 10 Gew.-% an B₂O₃, Bi₂O₃, ZnO, MgO, SnO₂, TiO₂, ZrO₂, La₂O₃ oder anderen Oxiden 3-wertiger Lanthanoide, K₂O, WO₃, GeO₂ sowie weiteren Zusätzen, welche die Eigenschaften nicht beeinträchtigen und physiologisch unbedenklich sind.

Besonders bevorzugt verwendete Pulver enthalten

| | |
|---|---|
| Si als SiO₂ | 25 - 45 Gew.-% |
| Al als Al₂O₃ | 20 - 40 Gew.-% |
| Ca als CaO | 10 - 30 Gew.-% |
| F | 10 - 30 Gew.-% |
| Na als Na₂O | 1 - 8 Gew.-% |
| P als P₂O₅ | 1 - 10 Gew.-%. |

Die erfindungsgemäß verwendeten Glaspulver weisen eine durchschnittliche Korngröße (Gewichtsmittel) von wenigstens 1 µm und bevorzugt mindestens 3 µm auf. Die durchschnittliche Korngröße (Gewichtsmittel) beträgt 1 - 20 µm, bevorzugt 3 - 15 µm und besonders bevorzugt 3 - 10 µm. Die Teilchen haben eine maximale Korngröße von 150 µm, vorzgusweise 100 µm, besonders 60 µm.

Die so erhaltenen Pulver werden dann gegebenenfalls einer Oberflächenbehandlung gemäß der europäischen Patentschrift 0 023 013 unterzogen. Hierzu werden die Glaspulver mit Säure oberflächlich behandelt, vorzgusweise beim Raumtemperatur. Dabei werden saure Gruppen enthaltende Substanzen eingesetzt, z.B. Salzsäure, Schwefelsäure, Salpetersäure, Essigsäure, Propionsäure oder Perchlorsäure, die lösliche Calciumsalze bzw. Strontiumsalze bilden. Die Säuren werden in einer Konzentration von 0,01 bis 10 Gew.-%, vorzugsweise von 0,05 bis 3 Gew.-% eingesetzt. Nach der entsprechenden Reaktionszeit werden die Pulver von der Lösung separiert und gründlich ausgewaschen, so daß sich praktisch keine löslichen Calcium- oder Strontiumsalze mehr auf der Oberfläche der Pulverteilchen befinden.

Die als Bestandteil (b) einzusetzenden Polysäuren können auch die bei der Herstellung von Glasionomerzementpulvern bekannten Polycarbonsäure, z.B. Polymaleinsäure, Polyacrylsäure, Polyitaconsäure sowie Gemisch davon oder Copolymere, insbesondere die aus der EP-B-0 024 056 bekannten Maleinsäure-Acrylsäure-Copolymere und/oder Acrylsäure-Itaconsäure-Copolymere sein. Das mittlere Molekulargewicht der erfindungsgemäß einzusetzenden Polycarbonsäuren beträgt mehr als 500. Vorteilhaft ist ein mittleres Molekulargewicht von 1.000 bis 20.000, besonders bevorzugt sind 3.000 bis 10.000. Die Polycarbonsäure wird vorzugsweise in Konzentrationen von 5 bis 50 Gew.-%, bezogen auf Bestandteil (a), eingesetzt.

Als Polysäure geeignet sind auch Polyphosphonsäuren, z.B. Polyvinylphosphonsäure. Diese Polyphosphonsäuren können die oben genannten Polysäuren ganz oder teilweise ersetzen.

Um hohe Lagerstabilitäten der Knochenersatzmaterialien vor der Anwendung zu erhalten, empfiehlt sich ein Zusatz von Konservierungsstoffen, z.B. Benzoesäure, insbesondere zur trockenen Polysäure.

Als Bestandteil (f) kann ein chelatbildendes Mittel, wie es in der DE-A-23 19 715 beschrieben ist, enthalten sein. Vorzgusweise wird als Chelatbildner Weinsäure eingesetzt.

Als schaumbildender Bestandteil (e) eignen sich alle Carbonate und/oder Hydrogencarbonate; vorzugsweise sind diese in der wässrigen Polysäurelösung, welche gegebenenfalls auch Chelatbildner enthält, zumindest teilweise löslich. Bevorzugt eingesetzt werden physiologische verträgliche Carbonate, wie die Carbonate und/oder Hydrogencarbonate der Alkali- und/oder Erdalkali-Metalle. Besonders bevorzugt sind die Carbonate und Hydrogencarbonate des Magnesiums, Calciums und Strontiums.

Die als schaumbildender Bestandteil (e) einsetzbaren Carbonate und/oder Hydrogencarbonate werden vorzugsweise in Konzentrationen von 0,1 bis 20 Gew.-%, bezogen auf Bestandteil (a), eingesetzt; bevorzugt eingesetzt werden 0,5 bis 5 Gew.-%, ganz besonders bevorzugt 1 bis 3 Gew.-%.

Daneben können auch andere Verschäumungsmittel eingesetzt werden, wobei auf den gesamten Inhalt der DE-A-39 27 984 verwiesen wird.

Als Verschäumunsmittel (e) eignen sich alle Materialien, die dazu geeignet sind, beim Herstellen der Formkörper eine Verschäumung zu bewirken. So ist es z.B. möglich, mit den flüssigen Bestandteilen (H₂O oder H₂O + Säure) unter Zusatz von Tensiden und/oder Emulgatoren und Einrühren von Gasen (z.B. Luft) stabile Schäume zu erzeugen, zu denen dann die anderen Bestandteile (z.B. Glas) hinzugefügt werden (siehe z.B. Ullmanns Enzyklopädie der technischen Chemie, Band 22, S. 463, Verlag Chemie, 4. Auflage, 1982). Weiter sind geeignet metallische Hydride, insbesondere Natriumborhydrid, welche mit Protonen (Wasser oder Säuren) unter Wasserstoffentwicklung eine Aufschäumung des härtenden Zements bewirken.

Eine weitere Möglichkeit der Schaumbildung besteht darin, eine wässrige Lösung eines Gases zu verwenden, z.B. von CO₂ oder SO₂, welches durch die zugegebene Säure beim Herstellen des Zements ausgetrieben wird und somit eine Schäumung bewirkt.

Möglich ist auch der Zusatz von Peroxiden, z.B. H₂O₂, die entweder durch Säureeinwirkung oder aber durch Metallkatalyse unter Zersetzung und Sauerstoffabgabe zu einem Verschäumen führen. So ist es beispielsweise möglich, eine wässrige H₂O₂-Lösung zum Anmischen des Zementes zu verwenden und in das Pulver eine entsprechende Menge FeSO₄ einzugeben, welches dann beim Zusammenbringen der beiden Komponenten zu einer Sauerstoffentwicklung und somit zu einer Aufschäumung führt.

Weitere Verschäumungsmittel sind Treibmittel, wie feste organische Treibmittel, z.B. Azodicarbonamid, Azobis-(isobutyronitril), Diphenyloxid-disulfonsäurehydrazid oder N-Nitroso-Verbindungen, feste anorganische Treibmittel, flüssige Treibmittel, z.B. Kohlenwasserstoffe oder Halogenkohlenwasserstoffe, und gasförmige Treibmittel, wie N₂, CO₂ und Luft.

Die festen und flüssigen Verschäumungsmittel werden üblicherweise in einer Konzentration von 0,01 - 50 Gew.-%, bezogen auf die Gesamtmischung, besonders bevorzugt 0,1 - 20 Gew.-%, eingesetzt, die gasförmigen Verschäumungsmittel werden in Mengen von 5 - 90 Vol.-%, bezogen auf die Gesamtmischung, vorzugsweise 10 - 60 Vol.-%, eingesetzt.

Das Gewichtsverhältnis von Komponente (b) zu Komponente (e) beträgt vorzugsweise mindestens 3:1, besonders bevorzugt ist ein Gewichtsverhältnis von mindetens 10:1.

Die Carbonate und/oder Hydrogencarbonate haben vorzugsweise eine mittlere Korngröße von 0,1 bis 200 µm, bevorzugt von 1 bis 100 µm, ganz besonders bevorzugt von 5 bis 50 µm.

Die Löslichkeit der Carbonate und/oder Hydrogencarbonate kann über die Wahl des (der) Kation(en)s gesteuert werden. Sie sollte so bemessen werden, daß der Schäumungsprozeß bis zur beginnenden Abbindung anhält. Bei einer gewünschten schnellen Abbindung empfiehlt sich somit die Wahl von leicht löslichen Alkalicarbonaten und/oder -hydrogencarbonaten, bei langsamer Abbindung die Wahl von schwerer löslichen Carbonaten und/oder Hydrogencarbonaten.

Für die erfindungsgemäßen Zwecke geeignete Zytostatika sind insbesondere Methotrexat, Vincristin, Cisplatin und Cyclophosphamid, und geeignete Antibiotika sind insbesondere die Gyrasehemmer, wie beispielsweise Ciprofloxacin, Ofloxacin, Norfloxacin und deren Salze sowie Aminoglycosid-Antibiotika, insbesondere die Klasse der Lincomycine. Ganz besonders bevorzugt sind Clindamycin und Lincomycin sowie deren Salze und Derivate. Auch die Kombination mehrerer Wirkstoffe kann geeignet sein, um spezielle Indikationsgebiete optimal zu versorgen. Bezüglich geeigneter Gyrasehemmer wird auf W. Stille, FAC Band 6-10, 1987, S. 1575-1583 verwiesen. Alle dort beschriebenen Verbindungen kommen in Betracht.

Die Konzentration der Zytostatika oder Antibiotika beträgt max. 10 Gew.-%, bevorzugt max. 3 Gew.-%, bezogen auf das Gesamtgewicht des Wirkstoffdepotmaterials. Besonders bevorzugt ist ein Bereich von 0,01 bis 3 Gew.-%. Bei höheren Dosen werden die mechanischen Eigenschaften des Glasionomerzements oder der daraus hergestellten Implantate beeinträchtigt.

### Beispiele

In allen Beispielen wird ein Calciumaluminiumfluorsilikatglaspulver mit der in Tabelle 1 wiedergegebenen oxidischen Zusammensetzung eingesetzt.

**Tabelle 1**

| | |
|---|---|
| Si als SiO₂ | 33,8 Gewichtsteile |
| Al als Al₂O₃ | 28,3 Gewichtsteile |
| Ca als CaO | 14,4 Gewichtsteile |
| Na als Na₂O | 2,6 Gewichtsteile |
| P als P₂O₅ | 6,7 Gewichtsteile |
| F | 17,3 Gewichtsteile |

### Beispiel 1

100 Gewichtsteile des Glaspulvers gemäß Tabelle 1 werden mit 1,1 Gewichtsteilen Clindamycin-Hydrochlorid (entsprechend 0,97 Teilen Clindamycin) homogen zu einer Pulvermischung I vermischt.

50 Gewichtsteile H₂O, 40 Gewichtsteile eines Copolymeren aus Acrylsäure und Maleinsäure (1:1, durchschnittliches Molekulargewicht 7000), 9,1 Gewichtsteile Weinsäure und 0,9 Gewichtsteile Benzoesäure werden zu einer homogenen Lösung I verrührt.

2,6 Gewichtsteile der Pulvermischung I und 1 Gewichtsteil der Lösung I werden homogen innerhalb einer halben Minute vermischt. Das Material hat eine Verarbeitungszeit von 4 Minuten. In dieser Zeit werden zylindrische Formkörper mit einem Durchmesser von 0,6 cm und einer Höhe von 1,2 cm hergestellt. 20 Minuten nach Herstellung werden diese in 5 ml Phosphatpuffer von pH 7,4 bei 37°C eingelegt und eluiert. Zu den angegebenen Zeitpunkten wurde die gesamte Elutionslösung ausgetauscht. Der Gehalt an Antibiotika im Eluat wurde im Bioassay als Agar-Diffusionstest bestimmt. Indikatorkeim für Clindamycin war S. aureus ATCC 25923. Undotierte Prüfkörper aus Glasionomerzement (ohne Zusatz an Clindamycin) zeigten keine Hemmhöfe.

**Tabelle 2**

| Freisetzung von Clindamycin aus Glasionomerzement | |
|---|---|
| Entnahmezeitpunkt in Tagen | Freisetzungsmenge in µg |
| 1 | 200 |
| 3 | 82 |
| 6 | 80 |
| 14 | 78 |
| 28 | 74 |
| 35 | 20 |

Die Wiederfindungsrate nach 35 Tagen beträgt ca. 12 % (kumulierte absolute Freisetzungsmenge).

Das Material ist als Knochenzement hervorragend geeignet. Es fließt sehr gut an Knochensubstanz an und hat gute adhesive Eigenschaften am Knochen. Die Druckfestigkeit des Zementes beträgt 123 MPa, die Biegefestigkeit 20 MPa.

### Beispiel 2

100 Gewichtsteile des in Tabelle 1 genannten Glases und 2,5 Gewichtsteile Clindamycin-Hydrochlorid (entsprechen 2,2 Teilen reinem Clindamycin) sowie 2 Gewichtsteile Calciumcarbonat (Firma Merck, mittlerer Korndurchmesser <40 µm) werden zu einer homogenen Pulvermischung II verarbeitet.

35 Gewichtsteile der in Beispiel 1 genannten Polycarbonsäure, 0,9 Gewichtsteile Benzoesäure und 64,1 Gewichtsteile destilliertes Wasser werden zu einer homogenen Lösung II verarbeitet.

2 Gewichtsteile der Pulvermischung II und 1 Gewichtsteil der Lösung II werden homogen innerhalb einer halben Minute vermischt.

Wie in Beispiel 1 beschrieben, werden zylindrische Formkörper hergestellt. Durch den Zusatz an Calciumcarbonat schäumt das Material auf und gibt einen offenporigen geschäumten Zement, der auch zu Granulaten verarbeitet werden kann. Das Material ist hervorragend geeignet zum Auffüllen von Knochendefekten und zum Zementieren von nichtdruckbelasteten Implantaten.

Wie in Beispiel 1 beschrieben, wurden die zylindrischen Prüfkörper in Phosphatpuffer von pH 7,4 eluiert und der Gehalt an Antibiotikum mittels Bioassay zu den in Tabelle 3 genannten Entnahmezeitpunkten gemessen.

**Tabelle 3**

| Freisetzung von Clindamycin aus geschäumten Glasionomerzementen | |
|---|---|
| Entnahmezeitpunkt in Tagen | Freisetzungsmenge in µg |
| 1 | 1800 |
| 2 | 120 |
| 7 | 190 |
| 14 | 40 |
| 28 | 25 |
| 35 | <10 |

Die Gesamtwiederfindungsrate beträgt 72 %. Das Material ist hervorragend geeignet als Wirkstoffdepot. Es gibt eine große Menge des Wirkstoffes bereits innerhalb der ersten 24 Stunden ab und sorgt innerhalb der darauffolgenden Wochen noch für eine kontinuierliche weitere Abgabe in geringen Dosen, um schließlich keinen meßbaren Gehalt an Antibiotikum abzugeben.

## Patentansprüche

1. Implantierbares Wirkstoffdepotmaterial, enthaltend einen Glasionomerzement und ein oder mehrere Zytostatika oder Antibiotika.

2. Wirkstoffdepotmaterial nach Anspruch 1, enthaltend
(a) ein Aluminiumfluorosilikatglas,
(b) mindestens eine Polysäure mit einem mittleren Molekulargewicht >500,
(c) gegebenenfalls Wasser,
(d) ein oder mehrere Zytostatika oder Antibiotika,
(e) gegebenenfalls ein Carbonat und/oder Hydrogencarbonat in einer Menge von mindestens 0,1 Gew.-%, bezogen auf (a), oder ein anderes Verschäumungsmittel, und
(f) gegebenenfalls ein chelatbildendes Mittel.

3. Wirkstoffdepotmaterial nach einem der Ansprüche 1 oder 2, dadurch gekennzeichnet, daß es als Antibiotika, Clindamycin und/oder Gyrasehemmer, in einer Menge von vorzugsweise 0,01 bis 3 Gew.-%, bezogen auf das Gesamtgewicht des Wirkstoffdepotmaterials, enthält.

4. Wirkstoffdepotmaterial nach den Ansprüchen 1 bis 3, dadurch gekennzeichnet, daß es in mindestens zwei räumlich voneinander getrennten Teilmassen vorliegt.

5. Wirkstoffdepotmaterial nach Anspruch 4, dadurch gekennzeichnet, daß mindestens eine Teilmasse in fester, vorzugsweise pulverförmiger Form und mindestens eine andere Teilmasse in flüssiger oder pastöser Form vorliegen.

6. Wirkstoffdepotmaterial nach Anspruch 5, dadurch gekennzeichnet, daß die feste Teilmasse die Bestandteile (a), (b) und (d) und die flüssige Teilmasse die Bestandteile (c) und (e) und gegebenenfalls den Bestandteil (f) enthalten.

7. Wirkstoffdepotmaterial nach Anspruch 5, dadurch gekennzeichnet, daß die feste Teilmasse die Bestandteile (a), (b), (d) und (e) und die flüssige Teilmasse die Bestandteile (c) und gegebenenfalls (f) enthalten.

8. Formkörper, erhältlich durch Härtung eines Wirkstoffdepotmaterials gemäß einem der Ansprüche 1 bis 7.

9. Verwendung eines Glasionomerzementes zur Herstellung von implantierbarem, ein oder mehrere Zytostatika oder Antibiotika enthaltendem Wirkstoffdepotmaterial.

10. Verwendung nach Anspruch 9, dadurch gekennzeichnet, daß das Wirkstoffdepotmaterial enthält:
(a) ein Aluminiumfluorosilikatglas,
(b) mindestens eine Polysäure mit einem mittleren Molekulargewicht >500,
(c) gegebenenfalls Wasser,
(d) ein oder mehrere Zytostatika oder Antibiotika,
(e) gegebenenfalls ein Carbonat und/oder Hydrogencarbonat in einer Menge von mindestens 0,1 Gew.-%, bezogen auf (a), oder ein anderes Verschäumungsmittel, und
(f) gegebenenfalls ein chelatbildendes Mittel.

## Claims

1. Implantable active substance depot material containing a glass ionomer cement and one or more cytostatics or antibiotics.

2. Active substance depot material according to claim 1, containing
(a) an aluminium fluorosilicate glass,
(b) at least one polyacid with an average molecular weight of >500,
(c) optionally water,
(d) one or more cytostatics or antibiotics,
(e) optionally a carbonate and/or hydrogen carbonate in an amount of at least 0.1% by weight based on (a), or another foaming agent, and
(f) optionally a chelating agent.

3. Active substance depot material according to one of claims 1 or 2, characterized in that it comtains as antibiotics clindamycin and/or gyrase inhibitor in an amount of preferably 0.01 to 3% by weight based on the total weight of the active substance depot material.

4. Active substance depot material according to claims 1 to 3, characterized in that it is present in at least two partial compositions which are spatially separated from each other.

5. Active substance depot material according to claim 4, characterized in that at least one partial composition is present in solid, preferably powdery form and at least one other partial composition is present in liquid or paste form.

6. Active substance depot material according to claim 5, characterized in that the solid partial composition contains constituents (a), (b) and (d) and the liquid partial composition contains constituents (c) and (e) and optionally (f).

7. Active substance depot material according to claim 5, characterized in that the solid partial composition contains constituents (a), (b), (d) and (e) and the liquid partial composition contains constituents (c) and optionally (f).

8. Molded structure obtainable by hardening an active substance depot material according to one of claims 1 to 7.

9. Use of a glass ionomer cement for producing implantable active substance depot material containing one or more cytostatics or antibiotics.

10. Use according to claim 9, characterized in that the active substance depot material contains:
(a) an aluminium fluorosilicate glass,
(b) at least one polyacid with an average molecular weight of >500,
(c) optionally water,
(d) one or more cytostatics or antibiotics,
(e) optionally a carbonate and/or hydrogen carbonate in an amount of at least 0.1% by weight based on (a), or another foaming agent, and
(f) optionally a chelating agent.

## Revendications

1. Matériau implantable pour dépôt de principe actif contenant un ciment de verre ionomère et un ou plusieurs cytostatiques ou antibiotiques.

2. Matériau pour dépôt de principe actif selon la revendication 1, contenant :
(a) un verre de fluorosilicate d'aluminium,
(b) au moins un polyacide ayant un poids moléculaire moyen > 500,
(c) éventuellement de l'eau,
(d) un ou plusieurs cytostatiques ou antibiotiques,
(e) éventuellement un carbonate et/ou un hydrogénocarbonate en une quantité d'au moins 0,1 % en poids par rapport à (a), ou un autre agent moussant, et
(f) éventuellement un agent chélateur.

3. Matériau pour dépôt de principe actif selon la revendication 1 ou 2, caractérisé en ce qu'il contient, comme antibiotique, de la clindamycine et/ou un inhibiteur de la gyrase, en une quantité de préférence de 0,01 à 3 % en poids par rapport au poids total du matériau pour dépôt de principe actif.

4. Matériau pour dépôt de principe actif selon les revendications 1 à 3, caractérisé en ce qu'il se présente sous la forme d'au moins deux masses partielles séparées spatialement lune de l'autre.

5. Matériau pour dépôt de principe actif selon la revendication 4, caractérisé en ce qu'au moins une masse partielle est à l'état solide, de préférence pulvérulent, et au moins une masse partielle est à l'état liquide ou pâteux.

6. Matériau pour dépôt de principe actif selon la revendication 5, caractérisé en ce que la masse partielle solide contient les constituants (a), (b) et (d), et la masse partielle liquide contient les constituants (c) et (e) et éventuellement le constituant (f).

7. Matériau pour dépôt de principe actif selon la revendication 5, caractérisé en ce que la masse partielle solide contient les constituants (a), (b), (d) et (e), et la masse partielle liquide contient les constituants (c) et éventuellement (f).

8. Article moulé qu'on peut obtenir par durcissement d'un matériau pour dépôt de principe actif selon l'une des revendications 1 à 7.

9. Utilisation d'un ciment de verre ionomère pour la fabrication d'un matériau implantable pour dépôt de principe actif contenant un ou plusieurs cytostatiques ou antibiotiques.

10. Utilisation selon la revendication 9, caractérisée en ce que le matériau pour dépôt de principe actif contient :
(a) un verre de fluorosilicate d'aluminium,
(b) au moins un polyacide ayant un poids moléculaire moyen > 500,
(c) éventuellement de l'eau,
(d) un ou plusieurs cytostatiques ou antibiotiques,
(e) éventuellement un carbonate et/ou un hydrogénocarbonate en une quantité d'au moins 0,1 % en poids par rapport à (a), ou un autre agent moussant, et
(f) éventuellement un agent chélateur.
